# EUROPÄISCHE PATENTSCHRIFT

(11) **EP 1 673 009 B1**
(45) Veröffentlichungstag und Bekanntmachung des Hinweises auf die Patenterteilung: **05.12.2012**
(21) Anmeldenummer: 04787003.5
(22) Anmeldetag: 24.09.2004
(51) Int. Cl.: A61B 5/022, G06F 17/00

(54) **BLUTDRUCK-MESSVERFAHREN UND BLUTDRUCKMESSGERÄT**
BLOOD PRESSURE MEASURING METHOD AND BLOOD PRESSURE MANOMETER
PROCEDE ET APPAREIL DE MESURE DE LA PRESSION ARTERIELLE

(30) Priorität: 26.09.2003 DE 10344803
(43) Veröffentlichungstag der Anmeldung: 28.06.2006
(73) Patentinhaber: Beurer GmbH, 89077 Ulm (DE)
(72) Erfinder: FORSTNER, Klaus, 71679 Asperg (DE)
(74) Vertreter: Fleck, Hermann-Josef
(86) Internationale Anmeldenummer: PCT/EP2004/010725
(87) Internationale Veröffentlichungsnummer: WO 2005/030050

(56) Entgegenhaltungen:
- EP-A- 0 850 592
- EP-A- 1 258 223
- US-A- 5 099 853
- US-A- 5 170 795
- US-A- 5 868 679
- DE MEERSMAN RONALD E ET AL: "Deriving respiration from pulse wave: A new signal-processing technique" AMERICAN JOURNAL OF PHYSIOLOGY, Bd. 270, Nr. 5 PART 2, 1996, Seiten H1672-H1675, XP008041228 ISSN: 0002-9513

## Beschreibung

Die Erfindung bezieht sich auf ein Blutdruck-Messverfahren, bei dem ein Pulsoszillogramm eines Patienten bestimmt und daraus der Blutdruck ermittelt und zur Anzeige gebracht wird, sowie ein Blutdruckmessgerät zur Durchführung des Verfahrens.

Ein Blutdruckmessverfahren bzw. ein Blutdruckmessgerät dieser Art ist in der EP-A 1 258 223 angegeben. Bei diesem bekannten Verfahren werden auf der Basis einer oszillometrischen Blutdruckmessung Blutdruckwerte erfasst, wobei festgestellt wird, ob bei der Blutdruckmessung hämodynamische Stabilität vorlag. Um die hämodynamische Stabilität zu beurteilen, werden mehrere Blutdruckmessungen durchgeführt, bei denen jeweilige Pulsoszillogramme aufgenommen werden.

Ein ähnliches nicht invasiv messendes Blutdruck-Messverfahren bzw. Blutdruckmessgerät ist in der EP 1 101 440 A1 angegeben. Bei diesem bekannten Verfahren bzw. Gerät, das auf einer oszillometrisch messenden automatischen Methode beruht, werden während eines Blutdruckmessvorganges wahlweise ein oder mehrere Pulsoszillogramme erzeugt, um aus diesem bzw. diesen die Blutdruckwerte zu ermitteln und zur Anzeige zu bringen. In der ersten Betriebsart werden in an sich üblicher Weise ein systolischer und ein diastolischer Blutdruckwert in einem Messzyklus mit einem einzigen Pulsoszillogramm ermittelt, während in der zweiten Betriebsart auf der Basis mehrerer bestimmter Pulsoszillogramme, zwischen denen eine Pause von 60 Sekunden eingehalten wird, u. a. festgestellt wird, ob eine sogenannte hämodynamische Stabilität vorliegt. Liegt keine hämodynamische Stabilität vor, wird dies dem Benutzer durch Ausgabe eines Fehlercodes angezeigt. Auf diese Weise wird der Benutzer also informiert, wenn die gemessenen Blutdruckwerte aufgrund ungenügender hämodynamischer Stabiität, insbesondere ungenügender Kreislaufruhe, verfälscht sind, wobei die Messzeit jedoch nicht unerheblich verlängert wird.

Ein in der DE 102 18 574 A1 gezeigtes Verfahren bzw. Gerät zum Messen des Blutdruckes ist zusätzlich zum Ermitteln einer Arrythmie ausgebildet, wozu eine Pulswelleninformation, wie die Breite, die Höhe und ein Zeitintervall, für eine Mehrzahl von Schlägen erfasst wird. Die Blutdruckwerte an sich können bei mangelnder Kreislaufruhe jedoch nicht genau gemessen werden.

Die mangelnde Kreislaufruhe gilt als der wichtigste Fehlereinfluss bei der ambulanten Messung des arteriellen Blutdruckes. Selbstmessende Patienten, aber auch medizinisches Fachpersonal, haben keine einfach zu erfassenden Kriterien bei der Blutdruckmessung, um Kreislaufruhe zu beurteilen. In vielen Fällen werden die Dauer und das Ausmaß der mangelnden Kreislaufruhe unterschätzt. Mangelnde Kreislaufruhe ist bei ärztlichen Messungen u. a. als sogenannter "white-coat-effect" dokumentiert und bekannt.

In der US-A 5,868,679 ist ein Blutdruckmessgerät mit einer aufblasbaren Manschette gezeigt, das insbesondere zur Dauerüberwachung von Patienten ausgebildet ist, wobei die Manschette wiederholt aufgeblasen wird und relativ niedrige Manschettendrücke angewendet werden.

Der Erfindung liegt die Aufgabe zugrunde, ein Blutdruck-Messverfahren bzw. Blutdruckmessgerät der eingangs genannten Art bereit zu stellen, mit dem ein Benutzer, insbesondere auch ein Laie, mit möglichst wenig Aufwand zuverlässige Blutdruckmessungen durchführen kann.

Diese Aufgabe wird mit den Merkmalen des Anspruches 1 bzw. des Anspruches 11 gelöst.

Bei dem Verfahren ist demnach vorgesehen, dass die Analyse bezüglich hämodynamischer Stabilität während der Bestimmung des individuellen Pulsoszillogramms durchgeführt wird, wobei mindestens ein hämodynamischer Parameter hinsichtlich zeitlicher Veränderungen ausgewertet wird, und dass aus der Analyse ein Beurteilungskriterium für das Vorliegen hämodynamischer Stabilität gewonnen wird, mit dem das Ermitteln des Blutdruckwertes oder der ermittelte Blutdruckwert in der Weise in Beziehung gebracht wird, dass festgestellt wird, ob der Blutdruckwert bei hämodynmaischer Stabilität erhalten wurde, oder dass ein korrigierter Blutdruckwert ermittelt wird. Das individuelle Pulsoszillogramm wird dabei der Analyse bezüglich der hämodynamischen Stabilität unterzogen. Im Einzelnen ist dabei vorgesehen, dass aus dem Pulsoszillogramm ein zeitabhängiger Pulsperiodenverlauf und/oder ein Pulsamplitudenverlauf und/oder die Pulsform ermittelt und analysiert wird/werden und dass das Beurteilungskriterium aus dem Pulsperiodenverlauf, aus dem Pulsamplitudenverlauf, aus der Pulsformänderung oder aus einer kombinierten Auswertung mindestens zweier dieser Basisinformationen gebildet wird, wobei ein besonders zuverlässiges Beurteilungskriterium erhalten wird, wenn die Basisinformationen in zumindest teilweiser Kombination miteinander in die Auswertung einbezogen werden.

Bei dem Blutdruckmessgerät ist vorgesehen, dass die Auswertevorrichtung des Weiteren eine Beurteilungseinrichtung aufweist, die so ausgebildet ist, dass mit ihr während der Bestimmung des individuellen Pulsoszillogramms ein Beurteilungskriterium für das Vorliegen hämodynamischer Stabilität gebildet wird, und dass die Anzeigevorrichtung mit einer Indikation für hämodynamische Instabilität versehen ist.

Mit diesen Maßnahmen wird erreicht, dass von einem Anwender ohne Mehraufwand, ohne Messzeitverlängerung sowie ohne zusätzliche Geräteeinstellungen erkannt wird, wenn eine Blutdruckmessung bei hämodynamischer Instabilität vorgenommen worden ist. Vorzugsweise werden dabei die Blutdruckwerte zusammen mit der Indikation der hämodynamischen Instabilität angezeigt, so dass beispielsweise auch Fachpersonal geeignete Rückschlüsse ziehen kann. Denkbar ist auch, dass lediglich die Tatsache des Fehlereinflusses signalisiert oder zu einer Wiederholungsmessung aufgefordert oder eine solche automatisch eingeleitet wird.

Zu einer benutzerfreundlichen Ausgestaltung trägt bei, dass mit dem Beurteilungskriterium eine Warnanzeige erzeugt wird, falls es von einem vorgegebenen oder vorgebbaren Schwellenkriterium abweicht, wobei auch die Art der Abweichung definiert vorgegeben werden kann.

Zur Erhöhung der Genauigkeit des Beurteilungskriteriums trägt bei, dass vor der Gewinnung des Beurteilungskriteriums Einflussgrößen aus Artefakten und/oder Arrythmien unterdrückt werden.

Dabei bestehen Ausgestaltungen darin, dass Pulsperiodendauern zumindest eines Anfangsbereichs und eines Endbereichs des Pulsoszillogramms miteinander verglichen werden und dass dem Beurteilungskriterium eine Abweichung der Pulsperiodendauern des Anfangsbereichs und des Endbereiches zugrundegelegt wird oder die Trendveränderung des Pulsperiodenverlaufs ermittelt wird.

Eine zum Vergleich mit einem Schwellenkriterium geeignete Größe besteht darin, dass die Abweichung der Pulsperiodendauern als Differenz der Periodendauern des Anfangsbereiches und des Endbereiches bezogen auf eine mittlere Pulsperiodendauer über das Pulsoszillogramm berechnet wird.

Weitere vorteilhafte Maßnahmen zur Beurteilung der hämodynamischen Stabilität bestehen darin, dass der gesamte Verlauf aller Pulsperioden bezüglich deren zeitlichen Änderung ermittelt wird und diese Änderung als ein Maß für die hämodynamische Stabilität herangezogen wird bzw. darin, dass der gesamte Verlauf der pulsspezifischen Systolenzeiten bezüglich deren zeitlichen Änderung ermittelt wird und diese Änderung als ein Maß für die hämodynamische Stabilität herangezogen wird.

Die Zuverlässigkeit des Beurteilungskriteriums wird dadurch verbessert, dass eine Bewertung der Stetigkeit insbesondere des gesamten zeitlichen Pulsperiodenverlaufs bei der Bildung des Beurteilungskriteriums mit einbezogen wird.

Weitere Maßnahmen, den Pulsamplitudenverlauf zur Bildung des Beurteilungskriteriums heranzuziehen, bestehen darin, dass aus dem Pulsamplitudenverlauf als charakteristische Größe(n) zum Bilden des Beurteilungskriteriums eine Steigung im ansteigenden Bereich der Einhüllenden oder eine Steigung in deren abfallendem Bereich oder eine Plateauweite um deren Maximum oder eine Kombination aus mindestens zweien dieser charakteristischen Größen herangezogen wird/werden.

Weiterhin lässt sich die Puls(kurven)form in der Weise auswerten, dass die Analyse der Pulsform eine Bestimmung einer oder mehrerer Steigungen an mindestens einem Punkt in einer ansteigenden und/oder in einer abfallenden Pulsflanke umfasst und dass als Beurteilungskriterium für die hämodynamische Stabilität eine zeitliche Änderung der Steigung(en) in den betreffenden Punkten oder ein Verhältnis der Steigungen in mindestens zwei Punkten eines Pulses für verschiedene Pulse untersucht wird.

Auf diese oder ähnliche Weise kann auch die Änderung der Systolenzeit als Beurteilungskriterium für die hämodynamische Stabilität ermittelt werden. Hierbei können z.B. je ein aussagekräftiger Basiswert im Fußbereich eines Pulses und im Spitzenbereich eines Pulses zum Bestimmen der Systolendauer zugrundegelegt werden. Diese Zeit ist mit der ventrikulären Kontraktionszeit des Herzens korreliert.

Ergeben sich aus dem Pulsperiodenverlauf und dem Pulsamplitudenverlauf z.B. unterschiedlich aussagekräftige charakteristische Größen, kann die Zuverlässigkeit zum Feststellen hämodynamischer Stabilität oder Instabilität dadurch verbessert werden, dass zum Bilden des Beurteilungskriteriums der Pulsperiodenverlauf, der Pulsamplitudenverlauf und/oder die Pulsform je nach Ausprägung gleich oder unterschiedlich gewichtet werden.

Zur Bewertung der hämodynamischen Stabilität besteht alternativ oder zusätzlich eine Ausgestaltungsmöglichkeit darin, dass als anderer bzw. additiver Parameter ein Atemfrequenzsignal, ein Elektrokardiogrammsignal und/oder ein Hautimpedanzmesssignal erfasst und hinsichtlich seiner zeitlichen Änderung während der individuellen Blutdruckmessung ausgewertet wird. Hierbei ist z.B. vorgesehen, dass das Atemfrequenzsignal aus der Analyse des Pulsoszillogramms oder mittels einer zusätzlichen Sensoranordnung gewonnen wird.

Für das Blutdruckmessgerät besteht eine vorteilhafte Ausgestaltung darin, dass die Beurteilungseinrichtung zum Erfassen eines Pulsperiodenverlaufes und/oder eines Pulsamplitudenverlaufs und/oder von Pulsformen aus dem Pulsoszillogramm und Bilden des Beurteilungskriteriums aus dem Pulsperiodenverlauf und/oder dem Pulsamplitudenverlauf und/oder einer Pulsformänderung ausgestaltet ist.

Eine alternative oder zusätzliche Ausgestaltungsmöglichkeit besteht darin, dass die Beurteilungseinrichtung zum Erfassen mindestens eines mit einer Änderung der Hämodynamik korrelierenden physiologischen additiven Parameters ausgebildet ist, der z.B. ein Atemfrequenzsignal, ein Elektrokardiogrammsignal und/oder ein Hautimpedanzmesssignal betrifft.

Die genannten Maßnahmen können beispielsweise in einem Oberarm- oder Handgelenk-Blutdruckmessgerät vorgesehen werden, wobei die Auswerte- und Anzeigevorrichtung in der Regel in einem Gehäuse auf der Manschette angeordnet sind, jedoch auch von der Manschette entfernt oder entfernbar angeordnet sein können. Die Blutdruckwerte können beispielsweise zusammen mit Datum und Uhrzeit und/oder Pulsfrequenz angezeigt und in einem geeigneten Speicher abgespeichert werden. Auch vorgegebene oder vorgebbare Grenzwerte können angezeigt, abgespeichert und überwacht werden. Auch kann an dem Gerät eine Schnittstelle zum Auslesen erfasster Daten und/oder Einlesen von Vorgabewerten oder Konfigurieren der Auswertevorrichtung vorgesehen sein.

Die Erfindung wird nachfolgend anhand von Ausführungsbeispielen unter Bezugnahme auf die Zeichnungen näher erläutert. Es zeigen:
- Fig. 1: typische Übergänge eines systolischen Blutdruckverlaufs und eines diastolischen Blutdruckverlaufs aus Bereichen hämodynamischer Instabilität in stationäre Bereiche in schematischer Darstellung,
- Fig. 2: eine schematische Darstellung eines Pulsoszillogramms mit der Einhüllenden,
- Fig. 3: eine schematische Darstellung zum Herleiten eines Beurteilungskriteriums für die hämodynamische Stabilität aus einem Pulsoszillogramm,
- Fig. 4A und 4B: Einhüllende verschiedener Pulsoszillogramme mit charakteristischen Größen in schematischer Darstellung,
- Fig. 4C: einen Pulskurvenverlauf und
- Fig. 5: eine weitere schematische Darstellung zur Herleitung eines Beurteilungskriteriums der hämodynamischen Stabilität.

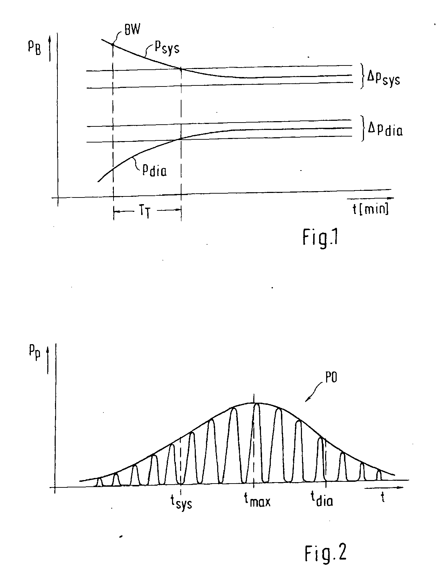

Fig. 1 veranschaulicht in einem Diagramm, in dem der Blutdruck p_{B} über der Zeit t aufgetragen ist, Übergangszeiten T_{T} eines systolischen Blutdruckverlaufs p_{sys} und eines diastolischen Blutdruckverlaufs p_{dia} aus einem Belastungswert BW in einen jeweiligen stationären Bereich Δp_{sys} bzw. Δp_{dia}. Die Werte Δp_{sys} und Ap_{dia} folgen aus der physiologischen Schlagvolumenvariation sowie kurzfristiger Gefäßweitenänderungen in ihrem Einfluss auf den Blutdruck.

Bewegen sich der systolische und der diastolische Blutdruck p_{sys}, p_{dia} sowie auch die Herzfrequenz eines Patienten um jeweils gültige stationäre Werte, also nicht auf einen Ruhewert zu oder von einem Ruhewert weg, liegt Kreislaufruhe vor. Kreislaufruhe ist Voraussetzung für die Gültigkeit international anerkannter Grenzwerte des arteriellen Blutdrucks (WHO, 1999 sowie JNC7, 2003). Diese Grenzwerte dienen als Zielgrößen bei der Einstellung eines arteriellen Blutdrucks.

Systolische und diastolische Blutdruckwerte ändern ihren Wert schlagweise. Dies ist die physiologische Kurzzeitvariation des arteriellen Blutdrucks. Sie kann typischerweise systolisch bis zu 12 mmHg und diastolisch bis zu 8 mmHg betragen. Neben diesen schlagbezogenen Veränderungen ist jedoch der Blutdruck des ruhenden, entspannten gesunden Menschen quasi stationär, d.h. nur sehr langsam veränderlich.

Kreislaufruhe liegt nicht mehr vor, wenn Menschen sich einer physischen Last unterziehen (müssen) oder sich einer psychischen Anspannung unterziehen (müssen). In diesen Fällen steigt in der Regel der systolische Blutdruck an, der diastolische Blutdruck fällt in der Regel in geringerem Ausmaße, kann aber auch ebenfalls ansteigen und die Pulsfrequenz erhöht sich regelhaft. Damit stellt sich jeder Organismus durch ein insgesamt höheres Herzschlagvolumen auf die entstandene Belastungssituation ein.

Nach Beendigung einer körperlichen oder psychischen Belastung benötigt der Organismus eine Übergangszeit T_{T} bis wieder Kreislaufruhe herrscht. Die Übergangszeit T_{T} ist von einer Reihe von Faktoren abhängig, insbesondere Höhe und Art der Belastung, Alter, Geschlecht, Trainingszustand, Vorerkrankungen.

Die Summe der genannten Faktoren kann in der Regel in ihrer Auswirkung auf die Ruhezeit nicht abgeschätzt werden. Insbesondere für den Laien ist es schwierig, Hinweise auf mangelnde Kreislaufruhe zu erhalten. In vielen Fällen wird deshalb in der Praxis die Übergangszeit T_{T} erheblich unterschätzt, so dass viele Blutdruckmessungen noch nicht in Kreislaufruhe erfolgen.

Typische Zeiten bis relative Kreislaufruhe erreicht ist (± 10 % von Ruhewerten) betragen 2 min bis 5 min. Bei älteren Menschen und vorerkrankten Patienten können Werte bis zu 15 min auftreten. Die Kreislaufruhe stellt aber den wichtigsten Fehlerfaktor bei der Bestimmung des Ruheblutdrucks eines Patienten dar und wird daher mit den im Folgenden näher beschriebenen Maßnahmen automatisch bei jedem individuellen Blutdruckmesszyklus diagnostiziert (hämodynamische Stabilitäts-Diagnostik = HSD). Ausgegangen wird dabei von einem Pulsoszillogramm PO, wie es beispielhaft in Fig. 2 dargestellt ist. Ein solches Pulsoszillogramm PO wird bei der vorliegend angewandten Methode der oszillometrischen Messung im Verlauf der Messung in an sich bekannter Weise stets erhoben.

Bei der vorliegenden hämodynamischen Stabilitäts-Diagnostik wird während eines Zyklus der oszillometrischen Blutdruckmessung geprüft, ob der betreffende Patient in hämodynamischer Ruhe ist oder nicht. Die Prüfung auf hämodynamische Stabilität führt zu einer Ergebnisanzeige, welche vorzugsweise den Zielgrößen systolischer Blutdruckwert, diastolischer Blutdruck und Pulsfrequenz beigeordnet wird. Die hämodynamische Stabilität wird dabei quantitativ ermittelt, dem Endbenutzer wird jedoch bevorzugt ein binärer Hinweis gegeben, ob die Stabilität als ausreichend angesehen wird oder nicht.

Der Benutzer hat zur Ermittlung der hämodynamischen Stabilität vor, während oder nach dem Messablauf keinerlei Tätigkeit oder Geräteeinstellung durchzuführen. Die Messzeit der Blutdruckmessung ist durch die hämodynamische Stabilitäts-Diagnostik nicht verlängert, da die Diagnose in demselben Messzyklus abläuft und die nachfolgende Signalanalyse praktisch unverzögert zu einer Ergebnisanzeige führt.

Die Bestimmung der hämodynamischen Stabilität gibt dem Ergebnis der oszillometrischen Blutdruckmessung die zusätzliche Information darüber, ob die notwendige Messbedingung zur Ermittlung des Ruheblutdrucks erfüllt war. Bei Nichteinhalten der Ruhebedingung kennzeichnet die hämodynamische Diagnose die gewonnenen Messungen als "Messung unter fehlender Kreislaufruhe" mit einer geeigneten Indikation.

Bei einem Pulsoszillogramm, wie es beispielsweise in Fig. 2 dargestellt ist, die einen Verlauf des Pulsdrucks p_{P} über der Zeit t zeigt, wächst die Amplitude der Einzelpulse während des Ablassens des Manschettendruckes bis zu einem Maximum an, das aufgrund physikalischer Gesetzmäßigkeiten dann erreicht ist, wenn der Manschettendruck dem mittleren arteriellen Blutdruck (MAP) entspricht. Anschließend nimmt die Amplitude der Einzelpulse wiederum ab. Der Amplitudenverlauf ist aus der ebenfalls eingezeichneten Einhüllenden ersichtlich.

Der systolische Blutdruck wird also in dem ansteigenden Abschnitt der Einhüllenden beispielsweise an einem Zeitpunkt t_{sys} und der diastolische Blutdruck in dem abfallenden Teil der Einhüllenden beispielsweise an einem Zeitpunkt t_{dia} erreicht. Diese Zeitpunkte ergeben sich durch geräteseitig vorgegebene Kalibrationskonstanten, die aus dem Pulsozillogramm abgeleitet werden. Dies gilt für Systole und Diastole. Aber auch bereits bevor die Manschette die abgedrückte Arterie wieder freigibt, wirken sich die herzseitig von der Manschette auftretenden Druckpulse auf den Manschettendruck aus (Anschlagpulse), so dass sich ein Oszillieren des Manschettendrucks und damit auch in dem Pulsoszillogramm bemerkbar macht, bevor der systolische Blutdruck p_{sys} beim Ablassen des Manschettendrucks erreicht ist. Dieser Effekt kann bei der Diagnose der hämodynamischen Stabilität mit verwendet werden.

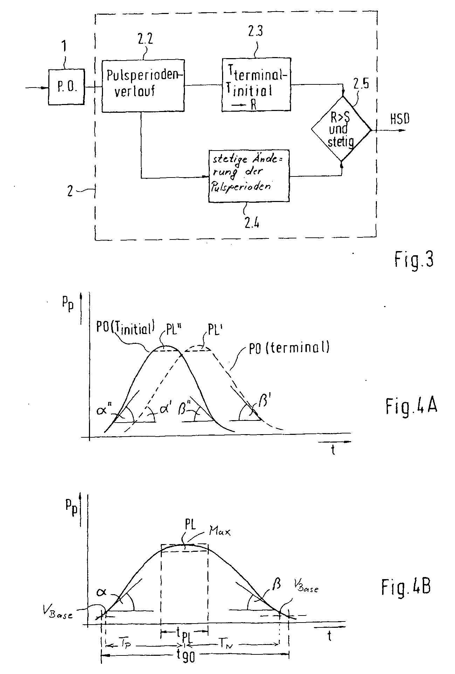

Für die Diagnose der hämodynamischen Stabilität werden gemäß Fig. 3, ausgehend von dem in einer Messstufe 1 gewonnenen Pulsoszillogramm in einer Pulsperioden-Sequenzanalyse 2 der Pulsperiodenverlauf in einer Auswertestufe 2.2 und aus diesem der Pulsabstand in einer Bestimmungsstufe 2.3 und die Stetigkeit der Änderung der Pulsperioden im Messverlauf in einer Ermittlungsstufe 2.4 ermittelt. Dabei wird in der Bestimmungsstufe 2.3 vorteilhaft der zeitliche Pulsabstand in einem anfänglichen Zeitabschnitt Tᵢₙᵢₜᵢₐₗ, der vor Erreichen des Maximums tₘₐₓ liegt und in einem späten Zeitabschnitt Tₜₑᵣₘᵢₙₐₗ gemessen und die Differenz der Pulsabstände Tₜₑᵣₘᵢₙₐₗ - Tᵢₙᵢₜᵢₐₗ durch eine Normierungsgröße, z.B. den mittleren Pulsabstand Tₘᵢₜₜₑₗ dividiert, um eine Bewertungsgröße R zu erhalten, die mit einer vorgegebenen oder vorgebbaren Schwelle S in einer Entscheiderstufe 2.5 verglichen wird. Als mittlerer Pulsabstand Tₘᵢₜₜₑₗ kann dabei z.B. der arithmetische Mittelwert aller erfassten Pulsabstände des Pulsoszillogramms PO zugrunde gelegt werden.

Außerdem wird der Entscheiderstufe 2.5 parallel zu dem Bewertungskriterium R in Form der Pulsperiodenänderung eine Stetigkeitsbewertung zugeführt, die in der Ermittlungsstufe 2.4 vorgenommen wird. In der Entscheiderstufe 2.5 wird dann aufgrund vorgegebener oder vorgebbarer Kriterien festgestellt, ob hämodynamische Stabilität während der Blutdruckmessung vorliegt oder nicht. Bereits aus dieser Pulsperioden-Sequenzanalyse kann auf das Vorliegen hämodynamischer Stabilität bzw. das Vorliegen stationärer Bedingungen geschlossen und eine entsprechende Indikation für die Anzeige erzeugt werden. Um eine möglichst große Zeitdifferenz zum Erfassen der anfänglichen und der späteren Pulsabstände Tᵢₙᵢₜᵢₐₗ und Tₜₑᵣₘᵢₙₐₗ und damit eine bessere Trennschärfe zu erhalten, ist es günstig, die anfänglichen Pulsabstände Tᵢₙᵢₜᵢₐₗ möglichst früh, also möglichst bereits die vor Erreichen des systolischen Drucks p_{sys} erhaltenen Impulse mit einzubeziehen, wie vorstehend erwähnt. Die späten Pulsabstände Tₜₑᵣₘᵢₙₐₗ sollten, soweit möglich, in einem späten Zeitbereich des abfallenden Pulsoszillogrammbereiches erfasst werden, welcher einen Bezug zum Zeitpunkt der diastolischen Druckbestimmung aufweist.

Eine Analyse des zeitlichen Verlaufs der Pulsperioden kann auf alle Pulse innerhalb einer Messung angewandt werden, indem deren zeitliche Änderung durch eine geeignete statistische Analyse - z.B. einer Regressionsanalyse - erfasst wird.

Eine weitere Aussage für das Vorliegen hämodynamischer Stabilität lässt sich mittels Auswertung der Pulsamplituden gewinnen, die insbesondere durch die Einhüllende des Pulsoszillogrammes PO gekennzeichnet und für verschiedene Fälle in den Fig. 4A und 4B dargestellt ist. Mit einer durchgezogenen Linie ist in Fig. 4A beispielhaft eine theoretische Einhüllende eines Pulsoszillogramms PO in einem anfänglichen Zeitabschnitt Tᵢₙᵢₜᵢₐₗ dargestellt. Eine gestrichelte Linie zeigt den Verlauf einer Einhüllenden in einem späten Zeitabschnitt Tₜₑᵣₘᵢₙₐₗ. Die verschiedenen Einhüllenden gehören zu stationären Kreislaufverhältnissen und zeigen als charakteristische Größen beispielsweise einen Anstiegswinkel *α'*, *α"* und einen Gefällwinkel *β', β"* und/oder (relative) Plateaubereiche PL', PL".

In Fig. 4B ist eine messtechnisch sich ergebende Einhüllende dargestellt, die als Summenkurve infolge der Überlagerung über den Messzeitraum zu Stande kommt. Der Summenkurve lassen sich ebenfalls entsprechende charakteristische Größen (*α*, *β*, PL) entnehmen, die wesentlich von der hämodynamischen Stabilität abhängen. Beispielsweise kann die Plateaudauer t_{PL} als Zeitraum bestimmt werden, in dem der Pulsdruck pₚ nicht weniger als ein vorgegebener prozentualer Wert (z.B. 10 %) unter dem Maximum liegt. Diese Plateaudauer kann zum Erhalten einer geeigneten Aussage auf eine weitere Zeitdauer bezogen werden, über die der Pulsdruck pₚ nicht weniger als ein niedrigerer vorgegebener prozentualer Wert (z.B. 90 %) unter dem Maximum liegt (z.B. t₉₀), so dass sich als charakteristische Größe z.B. R_{PL} = T_{PL} / T₉₀ ergibt.

Weiter kann die Anstiegszeit und die Abfallzeit von einem Wert V_{Base}, bezogen auf das Maximum für beide Flanken des Pulsoszillogramms, bestimmt werden. Es ergibt sich so die Abfallzeit T_{N} und die Anstiegszeit T_{P}. Die beiden Werte können in Beziehung zueinander gesetzt werden, z.B. durch einen Steilheitsindex S = T_{N}/T_{P}. Der Steilheitsindex S ändert sich während hämodynamischer Instabilität.

Die charakteristischen Größen nach den Fig. 4A und 4B können herangezogen werden, um den Pulsamplitudenverlauf zu charakterisieren und daraus Rückschlüsse auf das Vorliegen hämodynamischer Stabilität zu ziehen.

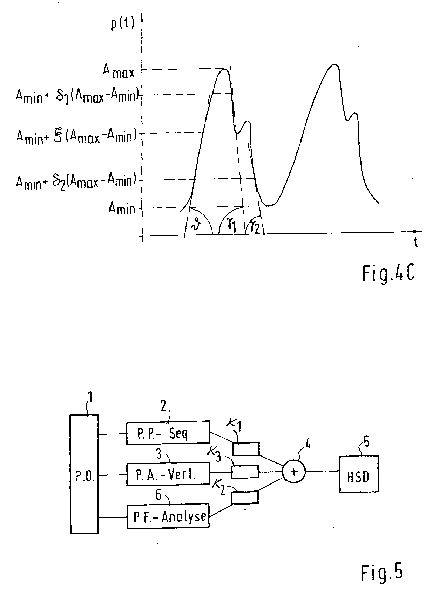

Ein weiteres Beurteilungskriterium für hämodynamische Stabilität ergibt sich aus einer Puls(kurven)formanalyse anhand charakteristischer Merkmale, z.B. gemäß Fig. 4C, die einen Pulskurvenverlauf p(t) über der Zeit t zeigt. Dabei werden als charakteristische Merkmale beispielsweise die Änderungen von Steilheiten an aufsteigenden und/oder abfallenden Pulsflanken im Messablauf bestimmt. In der aufsteigenden Pulsflanke wird die Steigung für einen Punkt *ξ* (Aₘₐₓ - Aₘᵢₙ) + Aₘᵢₙ berechnet, wobei Aₘₐₓ das Maximum und Aₘᵢₙ das Minimum der betreffenden Amplitude und *ξ* einen Wert zwischen null und eins bedeuten und die Steigung durch den Winkel *ϑ* gegeben ist. In der abfallenden Pulsflanke werden die Steigungen für die Punkte *δ*₁ (Aₘₐₓ - Aₘᵢₙ) + Aₘᵢₙ sowie *δ*₂ (Aₘₐₓ - Aₘᵢₙ) + Aₘᵢₙ berechnet, wobei *δ*₁ und *δ*₂ ebenfalls Werte zwischen null und eins sind und die Steigungen durch die Winkel *γ*₁, und *γ*₂ gegeben sind. Hämodynamische Veränderungen sind nun über die zeitlichen Änderungen der Steigungen *ϑ*, *γ*₁, und *γ*₂ erkennbar, so dass Rückschlüsse auf die hämodynamische Stabilität ermöglicht werden. Insbesondere sind z.B. die Verhältnisse aus *γ*₁/*ϑ* sowie *γ*₂/*ϑ* von diagnostischem Interesse.

In entsprechender oder ähnlicher Weise kann aus der Pulsform auch die Änderung einer Systolendauer ermittelt werden, beispielsweise zwischen einem im Fußbereich eines Pulses definierten charakteristischen Basiswert und einem im Bereich des Maximums definierten Spitzenwert. Aber auch der gesamte Verlauf der pulsspezifischen Systolenzeit kann einer Analyse, beispielsweise einer statistischen Trendanalyse, unterzogen werden. Auch die Systolendauer lässt sich für ein Beurteilungskriterium heranziehen.

Um eine möglichst hohe Zuverlässigkeit für die Bildung eines Beurteilungskriteriums zu erhalten, ob hämodynamische Stabilität während der Blutdruckmessung vorliegt oder nicht, können vorteilhaft mindestens zwei der Auswertungen Pulsperioden-Sequenzanalyse nach Fig. 3, die Pulsamplitudenanalyse und Pulsformanalyse in Kombination miteinander betrachtet werden, wie in Fig. 5 schematisch dargestellt.

Nach Fig. 5 werden, ausgehend von dem in der Messstufe 1 erhaltenen Pulsoszillogramm PO parallel die Pulsperioden-Sequenzanalyse 2, die Pulsamplitudenverlaufsanalyse 3 und die Pulsformanalyse 6 durchgeführt und beide Ergebnisse in einer Verknüpfungsstufe 4 miteinander verrechnet, um dann in einer Beurteilungsstufe 5 das Beurteilungskriterium, ob hämodynamische Stabilität vorliegt oder nicht, zu bilden. Vor oder in der Verknüpfungsstufe 4 oder in der Beurteilungsstufe 5 können dabei je nach charakteristischen Ausprägungen der Pulsperioden-Sequenzanalyse 2, der Pulsamplituden-Verlaufsanalyse 3 und/oder der Pulsformanalyse auch unterschiedliche Gewichtungen *κ*₁, *κ*₂, *κ*₃ dieser Analysen zum Bilden des Beurteilungskriteriums vorgenommen werden, wobei z.B. auch eine Kombination aus nur zwei dieser Analysen bzw. daraus gewonnener Aussagegrößen miteinander verknüpft werden können. Das Ergebnis, ob hämodynamische Stabilität festgestellt wird oder nicht, wird dann für die optische und/oder akustische Anzeige oder die automatische Durchführung einer Wiederholungsmessung verwendet, wobei im Falle nicht vorhandener hämodynamischer Stabilität eine entsprechende Warnanzeige bzw. Indizierung der Blutdruckwerte erfolgt. Auch ist eine Ausführungsform des Blutdruckmessverfahrens bzw. -geräts realisierbar, bei der das Ergebnis der hämodynamischen Stabilitätsanalyse zur Korrektur des Blutdruckwerts verwendet wird.

Vorzugsweise werden die genannten Verfahrensschritte bzw. Verarbeitungsstufen zum Beurteilen der hämodynamischen Stabilität softwaremäßig durch geeignete Programme in einem Mikrocontroller einer Auswertevorrichtung des Blutdruckmessgerätes verwirklicht. Dabei kann die Analyse des Pulsoszillogrammes zur Beurteilung der hämodynamischen Stabilität im Zeitbereich und/oder Frequenzbereich (spektrale Analyse) vorgenommen werden. Soweit zweckmäßig, können dabei geeignete Peripheriebausteine vorgesehen sein, um auch die Anzeige entsprechend zu steuern, gewünschtenfalls geeignete Werte abzuspeichern oder auch eine Schnittstelle für eine Ein-/Ausgabe zu steuern.

In der Auswertevorrichtung kann auch eine Auswahl von Parametersätzen vorgesehen sein, um z.B. Patienten-Manschetten automatisch zu erkennen oder andere Daten zu berücksichtigen. Auf der Grundlage der Parametersätze können dann auch im Einzelnen abgestimmte Programme ausgewählt werden, um eine entsprechend verfeinerte Diagnose der hämodynamischen Stabilität durchzuführen.

Auch ist es aufgrund charakteristischer Eigenschaften des Pulsperiodenverlaufs und/oder des Pulsamplitudenverlaufs und/oder der Pulsformanalyse denkbar, andere Einflussgrößen als die hämodynamische Instabilität als Einflussursachen für fehlerhafte Messwerte zu erkennen.

Bei einem weiteren Ausführungsbeispiel ist zur Beurteilung, ob bei der Messung des Blutdrucks hämodynamische Stabilität vorliegt oder nicht, vorgesehen, dass alternativ oder zusätzlich zu der vorstehend beschriebenen Analyse des individuellen Pulsoszillogramms PO während des Messzyklus ein oder mehrere physiologische additive bzw. weitere Paramter erfasst werden, die mit einer zeitlichen Änderung der Hämodynamik korrelieren. Derartige Sekundärparameter sind z.B. die Atmungsmodulation bzw. dieAtemfrequenz, ein Elektrokardiogrammsignal oderein Hautimpedanzsignal, das sich durch Variieren der Dehnung bei der Atmung oder des Feuchtigkeitszustandes ändert. Dabei kann die Atmungsmodulation z.B. bei der Analyse des bei der Blutdruckmessung erhobenen Pulsoszillogramms PO oder aber mittels einer zusätzlichen Sensoreinrichtung erfasst werden. Für die Gewinnung des Elektrokardiogrammsignals können Elektroden an der Manschette des Blutdruckmessgeräts angeordnet sein, während separat eine Gegenelektrode vorhanden ist. Durch Anschluss an das Blutdruckmessgerät, insbesondere an dessen Auswerteeinrichtung, können die Sekundärparameter bei der Gewinnung des Beurteilungskriteriums der hämodynamischen Stabilität bei vertretbarem Aufwand einbezogen werden. In ähnlicher Weise kann auch die absolute Pulsgeschwindigkeit beispielsweise über einen gesonderten Pulssensor erfasst und zur Beurteilung der hämodynamischen Stabilität berücksichtigt werden.

## Patentansprüche

1. Blutdruck-Messverfahren, bei dem ein Pulsoszillogramm (PO) eines Patienten bestimmt und daraus der Blutdruck (p_{B}) ermittelt und zur Anzeige gebracht wird, eine Analyse bezüglich hämodynamischer Stabilität unter Auswertung mindestens eines hämodynamischen Parameters hinsichtlich zeitlicher Veränderungen durchgeführt wird und aus der Analyse ein Beurteilungskriterium für das Vorliegen hämodynamischer Stabilität gewonnen wird, mit dem das Ermitteln des Blutdruckwertes oder der ermittelte Blutdruckwert in der Weise in Beziehung gebracht wird, dass festgestellt wird, ob der Blutdruckwert bei hämodynamischer Stabilität erhalten wurde, oder dass ein korrigierter Blutdruckwert ermittelt wird,
**dadurch gekennzeichnet,**
**dass** die Analyse bezüglich hämodynamischer Stabilität während der Bestimmung des individuellen Pulsoszillogramms (PO) in demselben Messzyklus wie die Blutdruckmessung durchgeführt wird, indem das individuelle Pulsoszillogramm (PO) der Analyse bezüglich der hämodynamischen Stabilität in der Weise unterzogen wird,
a) **dass** aus dem individuellen Pulsoszillogramm (PO) ein Pulsperiodenverlauf (2.2) ermittelt und Pulsperiodendauern zumindest eines Anfangsbereichs und eines Endbereichs des Pulsoszillogramms (PO) miteinander verglichen werden und dem Beurteilungskriterium eine Abweichung der Pulsperiodendauern des Anfangsbereiches (Tᵢₙᵢₜᵢₐₗ) und des Endbereichs (Tₜₑᵣₘᵢₙₐₗ) zugrunde gelegt wird oder der gesamte Verlauf aller Pulsperioden des Pulsoszillogramms (PO) bezüglich deren zeitlichen Änderung ermittelt wird und diese Änderung als ein Maß für die hämodynamische Stabilität herangezogen wird oder
b) **dass** ein Pulsamplitudenverlauf (3) ermittelt und aus diesem als charakteristische Größe(n) zum Bilden des Beurteilungskriteriums eine Steigung (α) im anfänglichen Bereich der Einhüllenden oder eine Steigung (β) in deren abfallendem Bereich oder eine Plateauweite (PL) um deren Maximum oder eine Kombination aus mindestens zweien dieser charakteristischen Größen herangezogen wird/werden, oder
c) **dass** die Pulsform (6) ermittelt und analysiert wird und die Analyse der Pulsform (6) eine Bestimmung einer oder mehrerer Steigungen an mindestens einem Punkt in einer ansteigenden und/oder in einer abfallenden Pulsflanke umfasst, wobei als Beurteilungskriterium für die hämodynamische Stabilität eine zeitliche Änderung der Steigung(en) in den betreffenden Punkten oder ein Verhältnis der Steigungen in mindestens zwei Punkten eines Pulses für verschiedene Pulse untersucht wird, oder
d) **dass** das Beurteilungskriterium aus zumindest zweien der Basisinformationen Pulsperiodenverlauf (2.2), Pulsamplitudenverlauf (3), Pulsformänderung aus einer kombinierten Auswertung gebildet wird.

2. Verfahren nach Anspruch 1,
**dadurch gekennzeichnet,**
**dass** mit dem Beurteilungskriterium eine Warnanzeige erzeugt wird und/oder automatisch eine Wiederholungsmessung eingeleitet wird, falls es von einem vorgegebenen oder vorgebbaren Schwellenkriterium abweicht.

3. Verfahren nach einem der vorhergehenden Ansprüche,
**dadurch gekennzeichnet,**
**dass** vor der Gewinnung des Beurteilungskriteriums Einflussgrößen aus Artefakten und/oder Arrythmien unterdrückt werden.

4. Verfahren nach einem der vorhergehenden Ansprüche,
**dadurch gekennzeichnet,**
**dass** die Abweichung der Pulsperiodendauern als Differenz der Periodendauern des Anfangsbereiches und des Endbereiches, bezogen auf eine mittlere Pulsperiodendauer, über das Pulsoszillogramm (PO) berechnet wird.

5. Verfahren nach einem der vorhergehenden Ansprüche,
**dadurch gekennzeichnet,**
**dass** der gesamte Verlauf der pulsspezifischen Systolenzeiten bezüglich deren zeitlichen Änderung ermittelt wird und diese Änderung als ein Maß für die hämodynamische Stabilität herangezogen wird.

6. Verfahren nach einem der vorhergehenden Ansprüche,
**dadurch gekennzeichnet,**
**dass** eine Bewertung der Stetigkeit des Pulsperiodenverlaufs (2.2) bei der Bildung des Beurteilungskriteriums mit einbezogen wird.

7. Verfahren nach einem der vorhergehenden Ansprüche,
**dadurch gekennzeichnet,**
**dass** zum Bilden des Beurteilungskriteriums der Pulsperiodenverlauf (2.2), der Pulsamplitudenverlauf (PA) und die Pulsform (6) je nach Ausprägung gleich oder unterschiedlich gewichtet werden.

8. Verfahren nach einem der vorhergehenden Ansprüche,
**dadurch gekennzeichnet,**
**dass** zusätzlich als ein anderer Parameter, der mit der hämodynamischen Stabilität korreliert, ein Atemfrequenzsignal, ein Elektrokardiogrammsignal und/oder ein Hautimpedanzmesssignal erfasst und hinsichtlich seiner zeitlichen Änderung während der individuellen Blutdruckmessung ausgewertet wird.

9. Verfahren nach Anspruch 8,
**dadurch gekennzeichnet,**
**dass** das Atemfrequenzsignal aus der Analyse des individuellen Pulsoszillogramms oder mittels einer zusätzlichen Sensoranordnung gewonnen wird.

10. Verfahren nach einem der vorhergehenden Ansprüche,
**dadurch gekennzeichnet,**
**dass** die Diagnostik einer hämodynamischen Instabilität zu einer automatisierten Korrektur des Fehlereinflusses herangezogen wird.

11. Blutdruckmessgerät zur Durchführung des Verfahrens nach Anspruch 1 mit einer aufblasbaren Manschette und einer darauf angeordneten oder daran anschließbaren Auswertevorrichtung mit einer ein Pulsoszillogramm (PO) erzeugenden Einheit (1), einer Blutdruck-Ermittlungseinrichtung, einer Beurteilungseinrichtung zur Ermittlung eines Beurteilungskriteriums für das Vorliegen hämodynamischer Stabilität und mit einer Anzeigevorrichtung, die mit einer Indikation für hämodynamische Instabilität versehen ist,
**dadurch gekennzeichnet,**
**dass** die Beurteilungseinrichtung zum Erfassen eines Pulsperiodenverlaufs (2.2) und/oder eines Pulsamplitudenverlaufs (3) und/oder von Pulsformen (6) aus dem Pulsoszillogramm (PO) in der Weise ausgebildet ist, dass mit ihr während der Bestimmung eines individuellen Pulsoszillogrammes (PO) in demselben Messzyklus wie die Blutdruckmessung das Beurteilungskriterium für das Vorliegen hämodynamischer Stabilität gebildet wird, wobei das Beurteilungskriterium aus dem Pulsperiodenverlauf (2.2) und/oder dem Pulsamplitudenverlauf (3) und/oder einer Pulsformänderung des individuellen Pulsoszillogramms (PO) dadurch gebildet wird,
a) **dass** aus dem individuellen Pulsoszillogramm ein Pulsperiodenverlauf (2.2) ermittelt und Pulsperiodendauern zumindest eines Anfangsbereichs und eines Endbereichs des Pulsoszillogramms (PO) miteinander verglichen werden und dem Beurteilungskriterium eine Abweichung der Pulsperiodendauern des Anfangsbereiches (Tᵢₙᵢₜᵢₐₗ) und des Endbereichs (Tₜₑᵣₘᵢₙₐₗ) zugrunde gelegt wird oder der gesamte Verlauf aller Pulsperioden des Pulsoszillogramms (PO) bezüglich deren zeitlichen Änderung ermittelt wird und diese Änderung als ein Maß für die hämodynamische Stabilität herangezogen wird oder
b) **dass** ein Pulsamplitudenverlauf (3) ermittelt und aus diesem als charakteristische Größe(n) zum Bilden des Beurteilungskriteriums eine Steigung (α) im anfänglichen Bereich der Einhüllenden oder eine Steigung (β) in deren abfallendem Bereich oder eine Plateauweite (PL) um deren Maximum oder eine Kombination aus mindestens zweien dieser charakteristischen Größen herangezogen wird/werden, oder
c) **dass** die Pulsform (6) ermittelt und analysiert wird und die Analyse der Pulsform (6) eine Bestimmung einer oder mehrerer Steigungen an mindestens einem Punkt in einer ansteigenden und/oder in einer abfallenden Pulsflanke umfasst, wobei als Beurteilungskriterium für die hämodynamische Stabilität eine zeitliche Änderung der Steigung(en) in den betreffenden Punkten oder ein Verhältnis der Steigungen in mindestens zwei Punkten eines Pulses für verschiedene Pulse untersucht wird, oder
d) **dass** das Beurteilungskriterium aus zumindest zweien der Basisinformationen Pulsperiodenverlauf (2.2), Pulsamplitudenverlauf (3), Pulsformänderung aus einer kombinierten Auswertung gebildet wird.

12. Blutdruckmessgerät nach Anspruch 11,
**dadurch gekennzeichnet,**
**dass** die Beurteilungseinrichtung zum Erfassen mindestens eines mit einer Änderung der Hämodynamik korrelierenden sekundären, anderen physiologischen Parameters ausgebildet ist, der ein Atemfrequenzsignal, ein Elektrokardiogrammsignal und/oder ein Hautimpedanzmesssignal betrifft.

## Claims

1. A blood pressure measurement method, in which a pulse oscillogram (PO) of a patient is determined, and from that the blood pressure (p_{B}) is ascertained and displayed, an analysis with regard to the hemodynamic stability is performed by evaluating at least one hemodynamic parameter with regard to changes over time, and from the analysis an assessment criterion for the presence of hemodynamic stability is obtained, with which criterion the ascertainment of the blood pressure value or the ascertained blood pressure value is put into relation in such a way that it is determined whether the blood pressure value was obtained at hemodynamic stability, or in such a way that a corrected blood pressure value is ascertained,
**characterized in that**
the analysis with regard to hemodynamic stability is performed during the determination of the individual pulse oscillogram (PO) in the same measurement cycle as the blood pressure measurement, **in that** the individual pulse oscillogram (PO) is subjected to the analysis with regard to the hemodynamic stability in such a way
a) that from the individual pulse oscillogram (PO), a pulse period course (2.2) is ascertained, and pulse period durations of at least one initial range and one terminal range of the pulse oscillogram (PO) are compared with one another, and a deviation in the pulse period durations of the initial range (Tᵢₙᵢₜᵢₐₗ) and of the terminal range (Tₜₑᵣₘᵢₙₐₗ) are made the basis of the assessment criterion, or the entire course of all the pulse periods of the pulse oscillogram (PO) is ascertained with regard to its change over time, and this change is used as a measure of the hemodynamic stability; or
b) that a pulse amplitude course (3) is ascertained and from it, a gradient (α) in the initial range of the envelope or a gradient (ß) in the descending range thereof or a plateau width (PL) about the maximum thereof, or a combination of at least two of these characteristic variables, is used as a characteristic variable or variables for forming the assessment criterion; or
c) that the pulse curve (6) is ascertained and analyzed, and the analysis of the pulse curve (6) includes a determination of one or more gradients at at least one point in an ascending and/or a descending pulse edge, and as the assessment criterion for the hemodynamic stability, a change over time in the gradient or gradients at the applicable points, or a ratio of the gradients at at least two points of one pulse is examined for various pulses; or
d) that the assessment criterion is formed from a combined evaluation of at least two of the basic items of information selected from the list comprising the pulse period course (2.2), the pulse amplitude course (3), and the change in the pulse curve.

2. The method according to claim 1,
**characterized in that**
with the assessment criterion, a warning display is generated and/or a repeat measurement is automatically initiated, if it deviates from a prespecified or prespecifiable threshold criterion.

3. The method according to one of the foregoing claims,
**characterized in that**
before the assessment criterion is obtained, influencing variables caused by artefacts and/or arrhythmias are suppressed.

4. The method according to one of the foregoing claims,
**characterized in that**
the deviation in the pulse period durations is calculated as the difference between the period durations of the initial range and the terminal range, referred to a mean pulse period duration, via the pulse oscillogram (PO),

5. The method according to one of the foregoing claims,
**characterized in that**
the entire course of the pulse-specific systolic times with regard to their change over time is ascertained, and this change is used as a measure for the hemodynamic stability.

6. The method according to one of the foregoing claims,
**characterized in that**
a weighting of the constancy of the pulse period course (2.2) is also included in the forming of the assessment criterion.

7. The method according to one of the foregoing claims,
**characterized in that**
for forming the assessment criterion, the pulse period course (2.2), the pulse amplitude course (PA), and the pulse curve (6) are weighted identically or differently, depending on their intensity.

8. The method according to one of the foregoing claims,
**characterized in that**
in addition, as another parameter which correlates with the hemodynamic stability, a respiratory rate signal, an electrocardiogram signal, and/or a skin impedance measurement signal is detected and evaluated with regard to its change over time during the individual blood pressure measurement.

9. The method according to claim 8,
**characterized in that**
the respiratory rate signal is obtained from the analysis of the individual pulse oscillogram or by means of an additional sensor array.

10. The method according to one of the foregoing claims,
**characterized in that**
the diagnosis of a hemodynamic instability is used for an automated correction of the influence of error.

11. A blood pressure measurement device for performing the method according to claim 1, having an inflatable cuff and an evaluation device with a unit (1) generating a pulse oscillogram (PO), the evaluation device being disposed on or connected to the cuff; having an assessment device for ascertaining an assessment criterion for the present of hemodynamic stability; and having a display device, which is provided with an indication for hemodynamic instability,
**characterized in that**
the assessment device for detecting a pulse period course (2.2) and/or a pulse amplitude course (3) and/or pulse curves (6) from the pulse oscillogram (PO) is embodied in such a way that with it, during the determination of an individual pulse oscillogram (PO), in the same measurement cycle as the blood pressure measurement, the assessment criterion for the presence of hemodynamic stability is formed, and the assessment criterion is formed from the pulse period duration (2.2) and/or the pulse amplitude course (3) and/or a change in the pulse curve of the individual pulse oscillogram (PO),
a) **in that** from the individual pulse oscillogram, a pulse period course (2.2) is ascertained, and pulse period durations of at least one initial range and one terminal range of the pulse oscillogram (PO) are compared with one another, and a deviation in the pulse period durations of the initial range (Tᵢₙᵢₜᵢₐₗ) and of the terminal range (Tₜₑᵣₘᵢₙₐₗ) are made the basis of the assessment criterion, or the entire course of all the pulse periods of the pulse oscillogram (PO) is ascertained with regard to its change over time, and this change is used as a measure of the hemodynamic stability; or
b) **in that** a pulse amplitude course (3) is ascertained and from it, a gradient (α) in the initial range of the envelope or a gradient (β) in the descending range thereof or a plateau width (PL) about the maximum thereof, or a combination of at least two of these characteristic variables, is used as a characteristic variable or variables for forming the assessment criterion; or
c) **in that** the pulse curve (6) is ascertained and analyzed, and the analysis of the pulse curve (6) includes a determination of one or more gradients at at least one point in an ascending and/or a descending pulse edge, and as the assessment criterion for the hemodynamic stability, a change over time in the gradient or gradients at the applicable points, or a ratio of the gradients at at least two points of one pulse is examined for various pulses; or
d) **in that** the assessment criterion is formed from a combined evaluation of at least two of the basic items of information selected from the list comprising the pulse period course (2.2), the pulse amplitude course (3), and the change in the pulse curve.

12. The blood pressure measurement device according to claim 11,
**characterized in that**
the assessment device for detecting at least one secondary, other physiological parameter correlating with a change in the hemodynamics is embodied, which relates to a respiratory rate signal, an electrocardiogram signal, and/or a skin impedance signal.

## Revendications

1. Procédé de mesure de la pression artérielle, dans lequel un oscillogramme de pouls (PO) d'un patient est établi et la pression artérielle (p_{B}) déterminée à partir de celui-ci et affichée, une analyse portant sur la stabilité hémodynamique est effectuée en évaluant au moins un paramètre hémodynamique en ce qui concerne ses variations dans le temps et un critère d'évaluation de la présence d'une stabilité hémodynamique est obtenu à partir de l'analyse, avec lequel la détermination de la valeur de pression artérielle ou la valeur de pression artérielle déterminée est mise en relation de façon qu'il soit établi si la valeur de pression artérielle a été obtenue en présence d'une stabilité hémodynamique ou qu'une valeur de pression artérielle corrigée soit déterminée,
**caractérisé en ce**
**que** l'analyse portant sur la stabilité hémodynamique est effectuée pendant la détermination de l'oscillogramme de pouls (PO) individuel dans le même cycle de mesure que la mesure de pression artérielle, l'oscillogramme de pouls (PO) individuel étant soumis à l'analyse portant sur la stabilité hémodynamique de telle sorte
a) **qu'**une courbe de période du pouls (2.2) est déterminée à partir de l'oscillogramme de pouls (PO) individuel, des durées de période du pouls d'au moins une zone initiale et une zone terminale de l'oscillogramme de pouls (PO) comparées entre elles et un écart des durées de période du pouls de la zone initiale (Tᵢₙᵢₜᵢₐₗ) et de la zone terminale (Tₜₑᵣₘᵢₙₐₗ) pris pour base du critère d'évaluation ou que la courbe totale de toutes les périodes du pouls de l'oscillogramme de pouls (PO) en ce qui concerne leur variation dans le temps est déterminée et cette variation utilisée comme une mesure de la stabilité hémodynamique ou
b) **qu'**une courbe d'amplitude du pouls (3) est déterminée et qu'à partir de celle-ci une pente (α) dans la zone initiale de l'enveloppe ou une pente (β) dans sa zone descendante ou une largeur de plateau (PL) autour de son maximum ou une combinaison d'au moins deux de ces grandeurs caractéristiques est utilisée comme grandeur(s) caractéristique(s) pour former le critère d'évaluation, ou
c) **que** la forme du pouls (6) est déterminée et analysée et l'analyse de la forme du pouls (6) comprend une détermination d'une ou plusieurs pentes à au moins un point sur un flanc d'impulsion montant et/ou descendant, une variation dans le temps de la pente (ou des pentes) aux points concernés ou un rapport des pentes à au moins deux points d'une impulsion pour différentes impulsions étant analysé(e) comme critère d'évaluation de la stabilité hémodynamique, ou
d) **que** le critère d'évaluation est formé à partir d'une évaluation combinée d'au moins deux des informations de base courbe de période du pouls (2.2), courbe d'amplitude du pouls (3), variation de forme du pouls.

2. Procédé selon la revendication 1,
**caractérisé en ce**
**qu'**un affichage d'avertissement est généré et/ou une mesure de répétition est déclenchée automatiquement avec le critère d'évaluation s'il diffère d'un critère seuil prédéfini ou prédéfinissable.

3. Procédé selon une des revendications précédentes,
**caractérisé en ce**
**que** les facteurs d'influence provenant d'artefacts et/ou d'arythmies sont éliminés avant l'obtention du critère d'évaluation.

4. Procédé selon une des revendications précédentes,
**caractérisé en ce**
**que** l'écart des durées de période du pouls est calculé sur l'oscillogramme de pouls (PO) en tant que différence des durées de période de la zone initiale et de la zone terminale, par rapport à une durée de période du pouls moyenne.

5. Procédé selon une des revendications précédentes,
**caractérisé en ce**
**que** la courbe totale des temps systoliques spécifiques du pouls en ce qui concerne leur variation dans le temps est déterminée et que cette variation est utilisée comme une mesure de la stabilité hémodynamique.

6. Procédé selon une des revendications précédentes,
**caractérisé en ce**
**qu'**une évaluation de la continuité de la courbe de période du pouls (2.2) est prise en compte dans la formation du critère d'évaluation.

7. Procédé selon une des revendications précédentes,
**caractérisé en ce**
**que** pour former le critère d'évaluation, la courbe de période du pouls (2.2), la courbe d'amplitude du pouls (PA) et la forme du pouls (6) sont pondérées de manière égale ou différente selon leur caractérisation.

8. Procédé selon une des revendications précédentes,
**caractérisé en ce**
**qu'**un autre paramètre qui est corrélé à la stabilité hémodynamique, un signal de fréquence respiratoire, un signal d'électrocardiogramme et/ou un signal de mesure d'impédance de la peau, est détecté en plus et évalué en ce qui concerne sa variation dans le temps pendant la mesure de pression artérielle individuelle.

9. Procédé selon la revendication 8,
**caractérisé en ce**
**que** le signal de fréquence respiratoire est obtenu à partir de l'analyse de l'oscillogramme de pouls individuel ou au moyen d'un dispositif de détection supplémentaire.

10. Procédé selon une des revendications précédentes,
**caractérisé en ce**
**que** le diagnostic d'une instabilité hémodynamique est utilisé pour une correction automatisée du facteur d'erreur.

11. Appareil de mesure de la pression artérielle pour réaliser le procédé selon la revendication 1, avec un brassard gonflable et un dispositif d'évaluation disposé dessus ou pouvant y être raccordé, avec une unité (1) qui génère un oscillogramme de pouls (PO), un dispositif de détermination de la pression artérielle, un dispositif d'évaluation de la présence d'une stabilité hémodynamique et avec un dispositif d'affichage qui est pourvu d'une indication d'une instabilité hémodynamique, **caractérisé en ce**
**que** le dispositif d'évaluation est conçu pour détecter une courbe de période du pouls (2.2) et/ou une courbe d'amplitude du pouls (3) et/ou une forme du pouls (6) à partir de l'oscillogramme de pouls (PO) de façon que celle-ci soit utilisée pour former le critère d'évaluation de la présence d'une stabilité hémodynamique pendant la détermination d'un oscillogramme de pouls (PO) individuel dans le même cycle de mesure que la mesure de pression artérielle, le critère d'évaluation étant formé à partir de la courbe de période du pouls (2.2) et/ou de la courbe d'amplitude du pouls (3) et/ou d'une variation de forme du pouls de l'oscillogramme de pouls (PO) individuel par le fait
a) **qu'**une courbe de période du pouls (2.2) est déterminée à partir de l'oscillogramme de pouls individuel, des durées de période du pouls d'au moins une zone initiale et une zone terminale de l'oscillogramme de pouls (PO) sont comparées entre elles et un écart des durées de période du pouls de la zone initiale (Tᵢₙᵢₜᵢₐₗ) et de la zone terminale (Tₜₑᵣₘᵢₙₐₗ) pris pour base du critère d'évaluation ou que la courbe totale de toutes les périodes du pouls de l'oscillogramme de pouls (PO) en ce qui concerne leur variation dans le temps est déterminée et cette variation utilisée comme une mesure de la stabilité hémodynamique ou
b) **qu'**une courbe d'amplitude du pouls (3) est déterminée et qu'à partir de celle-ci une pente (α) dans la zone initiale de l'enveloppe ou une pente (β) dans sa zone descendante ou une largeur de plateau (PL) autour de son maximum ou une combinaison d'au moins deux de ces grandeurs caractéristiques est utilisée comme grandeur(s) caractéristique(s) pour former le critère d'évaluation, ou
c) **que** la forme du pouls (6) est déterminée et analysée et l'analyse de la forme du pouls (6) comprend une détermination d'une ou plusieurs pentes à au moins un point sur un flanc d'impulsion montant et/ou descendant, une variation dans le temps de la pente (ou des pentes) aux points concernés ou un rapport des pentes à au moins deux points d'une impulsion pour différentes impulsions étant analysé(e) comme critère d'évaluation de la stabilité hémodynamique, ou
d) **que** le critère d'évaluation est formé à partir d'une évaluation combinée d'au moins deux des informations de base courbe de période du pouls (2.2), courbe d'amplitude du pouls (3), variation de forme du pouls.

12. Appareil de mesure de la pression artérielle selon la revendication 11, **caractérisé en ce**
**que** le dispositif d'évaluation est conçu pour détecter au moins un autre paramètre physiologique secondaire, corrélé à une variation de l'hémodynamique, qui concerne un signal de fréquence respiratoire, un signal d'électrocardiogramme et/ou un signal de mesure d'impédance de la peau.
